Europäisches Patentamt

European Patent Office

Office européen des brevets

(11) Veröffentlichungsnummer: **0 184 247 B1**

(19)

(12) **EUROPÄISCHE PATENTSCHRIFT**

(45) Veröffentlichungstag der Patentschrift: **28.08.91**

(21) Anmeldenummer: **85201855.5**

(22) Anmeldetag: **12.11.85**

(51) Int. Cl.⁵: **G01N 23/203**, A61B 6/00,
A61B 6/06

(54) **Anordung zur Untersuchung eines Körpers mit Gamma- oder Röntgenstrahlung.**

(30) Priorität: **27.11.84 DE 3443095**

(43) Veröffentlichungstag der Anmeldung:
**11.06.86 Patentblatt 86/24**

(45) Bekanntmachung des Hinweises auf die
Patenterteilung:
**28.08.91 Patentblatt 91/35**

(84) Benannte Vertragsstaaten:
**DE FR GB IT SE**

(56) Entgegenhaltungen:
**EP-A- 0 074 021**
**US-A- 3 539 808**

(73) Patentinhaber: **Philips Patentverwaltung
GmbH
Wendenstrasse 35 Postfach 10 51 49
W-2000 Hamburg 1(DE)**

(84) Benannte Vertragsstaaten:
**DE**

Patentinhaber: **N.V. Philips' Gloeilampenfabrieken
Groenewoudseweg 1
NL-5621 BA Eindhoven(NL)**

(84) Benannte Vertragsstaaten:
**FR GB IT SE**

(72) Erfinder: **Harding, Geoffrey, Dr.
Tönninger Weg 45
W-2000 Hamburg 52(DE)**

(74) Vertreter: **Hartmann, Heinrich, Dipl.-Ing. et al
Philips Patentverwaltung GmbH Wendenstrasse 35 Postfach 10 51 49
W-2000 Hamburg 1(DE)**

## Beschreibung

Die Erfindung betrifft eine Anordnung nach dem Oberbegriff des Anspruchs 1. Eine solche Anordnung ist aus der DE-PS 27 13 581 bekannt. Der Schlitz bildet dabei den vom Primärstrahl durchsetzten Bereich auf der (ortsauflösenden) Detektoranordnung ab, so daß jeder Bereich auf dem Primärstrahl einem bestimmten räumlichen Bereich der Detektoranordnung zugeordnet ist. Auf diese Weise kann die Streudichteverteilung längs des Primärstrahls mittels einer einzigen Messung erfaßt werden. Zur Erfassung der Streudichteverteilung in einem zwei- oder dreidimensionalen Bereich muß eine Relativverschiebung zwischen der Strahlenquelle und der Blenden- und Detektoranordnung einerseits und dem zu untersuchenden Körper andererseits in einer oder zwei zur Richtung des Primärstrahls senkrechten Richtung erfolgen. Die Erfassung der Streudichte in einem mehrdimensionalen Bereich erfordert daher relativ viel Zeit.

Aufgabe der Erfindung ist es, eine Anordnung der eingangs genannten Art so auszugestalten, daß sich bei kompakter Bauweise eine Verkürzung der Untersuchungszeiten ergibt. Der sich drehende Blendenteil bewirkt eine Verschiebung des Primärstrahles, ohne daß die Blenden- und die Detektoranordnung einerseits und der zu untersuchende Körper andererseits relativ zueinander verschoben werden müssen. Dies gestattet eine schnelle Abtastung des Körpers, ohne daß Erschütterungen auftreten.

Da die zwischen der Strahlenquelle und dem Körper befindliche Blendenanordnung und die Detektoranordnung sich in einem gemeinsamen Gehäuse befinden, kann die Detektoranordnung nur die aus dem Körper rückwärts gestreute Streustrahlung erfassen. Die Auswertung der rückwärts gestreuten Streustrahlung erlaubt einerseits eine kompakte Bauform (mit Blenden- und Detektoranordnung im gleichen Gehäuse) und gestattet andererseits auch die Untersuchung von Objekten, die nur von einer Seite zugänglich sind.

Eine Weiterbildung der Erfindung sieht vor, daß die Blendenanordnung mehrere Öffnungen für mehrere Primärstrahlen enthält und daß jedem Primärstrahl wenigstens je ein Schlitz und je eine Detektoranordnung zur Erfassung der durch die Öffnung hindurchtretenden Primärstrahl erzeugten Streustrahlung zugeordnet sind. Dabei können mehrere Primärstrahlen gleichzeitig den Körper abtasten, was zu einer weiteren Verkürzung der Untersuchungszeit führt.

Die Erfindung wird nachstehend anhand der Zeichnung näher erläutert. Es zeigen

Fig. 1 einen Querschnitt durch eine erfindungsgemäße Anordnung und

Fig. 2 eine Seitenansicht eines Teils dieser Anordnung.

Eine annähernd punktförmige Strahlenquelle 1 in Form beispielsweise eines im übrigen nicht näher dargestellten Röntgenstrahlers emittiert ein in der Zeichenebene und senkrecht dazu ausgedehntes Strahlenbündel, das auf eine Blenden- und Meßeinrichtung 2 trifft, die aus dem Strahlenbündel drei Primärstrahlen 3, 4, 5 mit geringem Querschnitt ausblendet. Diese drei divergierenden Primärstrahlen durchqueren das zu untersuchende Objekt 6 beispielsweise ein Werkstück, das sich auf einer dünnen, die Strahlung nur wenig absorbierenden Platte 7 befindet.

Die Blenden- und Meßeinrichtung umfaßt einen plattenförmigen Oberteil 8 und einen quaderförmigen Unterteil 9, in dem drei Hohlwalzen 30, 40 und 50 um ihre Längsachsen drehbar angeordnet sind. Diese Längsachsen liegen parallel zueinander in einer horizontalen zur Zeichenebene senkrechten Ebene. Die Längsachsen verlaufen nicht genau senkrecht zur Zeichenebene, wobei die Abweichungen vom senkrechten Verlauf durch die Divergenz der Strahlung bestimmt sind. Jede der drei Hohlwalzen weist zwei auf ihrem Umfang um 180° gegeneinander versetzte schmale Öffnungen 31 und 32, 41 und 42; bzw. 51 und 52 auf, die sich konisch zur Walzenmitte verbreitern, um etwaige Strahlabschattungen in den Randbereichen aufgrund der Strahlendivergenz zu vermeiden.

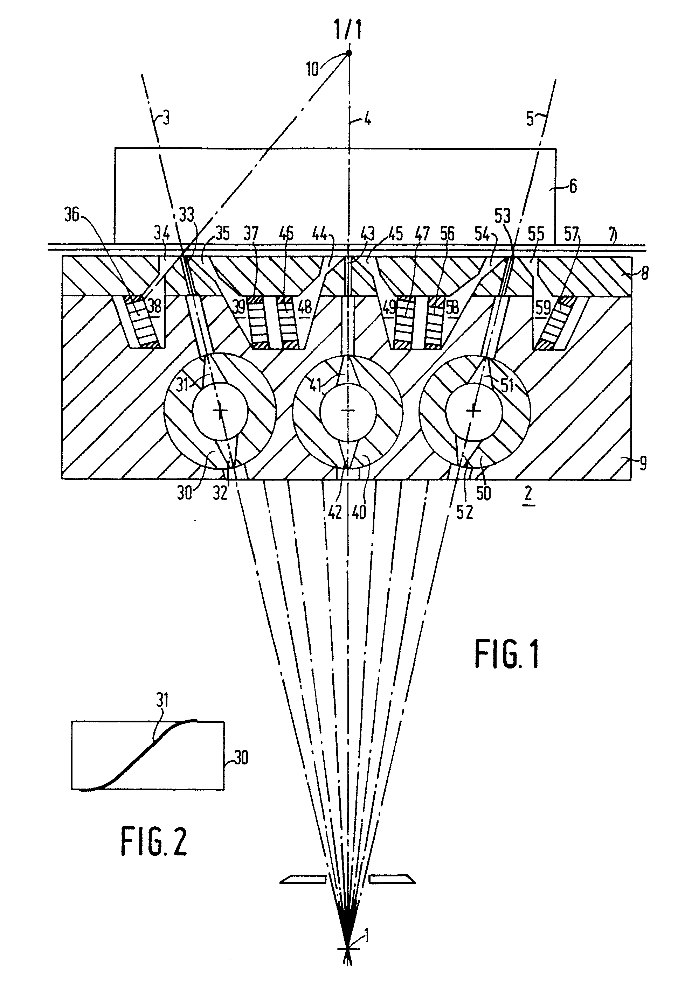

Wie aus Fig. 2 hervorgeht, die die Walze 30 in einer Seitenansicht zeigt, ist die Öffnung 31 Teil eines spiralförmig durch den Walzenmantel verlaufenden Schlitzes. Das gleiche gilt auch für alle anderen Öffnungen, wobei die beiden spiralförmigen Schlitze auf jeder Walze um genau 180° versetzt sind. In jeder Winkelstellung der Walze (bezogen auf ihre Drehachse) gibt es daher eine Stelle auf der Längsachse, durch die hindurch die von der Strahlenquelle 1 emittierte Strahlung den oberen und den unteren spiralförmigen Schlitz passieren kann, so daß hinter der Walze (bezogen auf die Strahlenquelle) ein (primär-) Strahl mit geringem Querschnitt zur Verfügung steht, der auch den Unterteil passieren kann, da dieser in einem relativ großen Bereich beiderseits einer die Walzenachse und die punktförmige Strahlenquelle 1 enthaltenden Ebene mit einer schlitzförmigen Öffnung versehen ist. Die auf diese Weise erzeugten Primärstrahlen 3, 4 und 5 passieren den Oberteil 8 durch schmale schlitzförmige Öffnungen 33, 43 bzw. 53 hindurch, die sich senkrecht zur Zeichenebene erstrecken. Die Primärstrahlen erzeugen in dem zu untersuchenden Körper 6 Streustrahlung, die sich um den Primärstrahl herum nach vorne, zur Seite und nach rückwärts ausbreitet. Die rückgestreute Strahlung gelangt durch Schlitze 34, 35; 44, 45; 54, 55 auf jeweils eine ortsauflösende Detektoranordnung 36, 37; 46, 47; 56, 57, die sich in je einer Kammer 38, 39; 48, 49; bzw. 58, 59 befindet. Je zwei Kammern und die darin befindlichen Detektoren befinden sich beiderseits einer einen der Primärstrahlen enthaltenden zur Zeichenebene senkrechten Ebene, so daß die darin enthaltenen Detektoranordnungen im wesentlichen nur die Streustrahlung des diese Ebene jeweils bestimmenden Primärstrahls erfassen - z.B. die Detektoranordnungen 36 und 37, die Streustrahlung im Primärstrahl 3, die Detektoranordnungen 46 und 47, die Streustrahlung im Primärstrahl 4 usw.

Querschnitte durch die Anordnung 8, 9 und die darin enthaltenen Teile in zur Zeichenebene parallelen Ebenen sind identisch - wenn man einmal davon absieht, daß dies nicht für die Ablenkeinrichtung mit den Walzen gilt, die nicht genau senkrecht zur Zeichenebene verlaufen.

Jede Detektoranordnung besteht aus mehreren Detektoren - z.B. Kristalldetektoren - und ist zusammen mit dem dazugehörigen Schlitz so angeordnet, daß ein Abschnitt des Primärstrahls durch den Schlitz auf die Detektoranordnung abgebildet wird, wie das in der DE-PS 27 13 581 beschrieben ist. Im vorliegenden Fall ist diese Anordnung so getroffen, daß nur die Streustrahlung erfaßt werden kann, die in einem unteren, etwa bei der Platte 7 beginnenden und in einigem Abstand davon endenden Abschnitt erfaßt werden kann. Dies führt dazu, daß bei einem dickeren Körper die Streudichte in einem oberen Abschnitt nicht mehr erfaßt werden kann, doch schadet dies nicht, wenn es nur darum geht, Inhomogenitäten in dem unteren Abschnitt zu erfassen.

Diese Anordnung der Schlitze (z.B. 34) und der zugehörigen Detektoranordnung (z.B. 36) bringt es aber mit sich, daß die Detektoranordnung durch den Schlitz hindurch auch einen benachbarten Primärstrahl (z.B. 4) "sehen" kann. Streustrahlung, die durch den primärstrahl 4 im Punkt 10 erzeugt würde, könnte also durch den Schlitz 34 hindurch den obersten Detektor bzw. den obersten Detektorabschnitt der Detektoranordnung 36 erreichen und die Messung insoweit verfälschen, als bei Rekonstruktion davon ausgegangen wird, daß die von der Detektoranordnung 36 erfaßte Streustrahlung ausschließlich im Bereich des Primärstrahls 3 erzeugt wird.

Jedoch ist diese Verfälschung sehr gering, weil - vorausgesetzt, daß der Körper überhaupt so dick ist, daß sich im Punkt 10 ein Streuzentrum befindet - die von diesem Punkt ausgehende streustrahlung weitgehend durch den Körper 6 absorbiert wird, wobei noch hinzukommt, daß der Primärstrahl 4 bereits geschwächt ist, bevor er den Punkt 10 erreicht. Die vom Punkt 10 ausgehenden die Detektoranordnung 36 erreichende Streuintensität ist daher vernachlässigbar klein. Dies wird zum einen dadurch bewirkt, daß die rückgestreute Strahlung erfaßt, zum anderen aber dadurch, daß der Winkel zwischen benachbarten primärstrahlen 3 und 4 oder 4 und 5 relativ groß ist (etwa 15°) und daß jeweils nur die Streustrahlung im unteren Abschnitt, die durch den Körper 6 relativ wenig geschwächt wird, erfaßt wird. Die Tatsache, daß lediglich die rückgestreute Strahlung erfaßt wird, führt darüber hinaus zu einer kompakten Bauweise, weil, wie aus der der Zeichnung ersichtlich, die Blendenanordnungen 30, 33...50, 53 und die Dekektoranordnungen 36, 37...56, 57 baulich miteinander kombiniert werden können.

Um mit jedem Primärstrahl 3, 4, 5 einen zweidimensionalen Bereich abtasten zu können, ist es erforderlich, das Objekt 6 und den Primärstrahl relativ zueinander zu verschieben. Dies kann in einfachster Weise durch Drehung der Walzen 30, 40 und 50 um jeweils denselben Betrag um ihre Achsen erfolgen. In diesem Fall verschiebt sich die Stelle, an der Strahlung durch die beiden schlitzförmigen Öffnungen einer Walze hindurchtreten kann senkrecht zur Zeichenebene, was zur Folge hat, daß die durch die Primärstrahlen 3, 4 und 5 definierte Ebene um den durch die Strahlenquelle 1 definierten Punkt geschwenkt wird.Ein dreidimensionaler Bereich wird erfaßt, wenn das Objekt 6 einerseits und die Blenden- und Detektoranordnung zusammen mit der Strahlenquelle 1 andererseits relativ zueinander in horizontaler Richtung verschoben werden.

Bei der in der Zeichnung dargestellten Ausführungsform wird die Verschiebung der Primärstrahlen 3, 4 und 5 senkrecht zur Zeichenebene dadurch erreicht, daß die drei Walzen, die aus der EP-A-0 074 021 an sich bekannt sind, jeweils um einen bestimmten Winkelbetrag gedreht werden. Anstelle der Walzen könnte aber auch eine um eine vertikale Achse drehbare mit einer zu den schlitzförmigen Öffnungen 33, 43 und 53 etwa senkrecht verlaufenden schlitzförmigen Öffnung versehene Blendenplatte verwendet werden, wie dies aus der DE-OS 30 32 801 an sich bekannt ist. In diesem Fall wird also die Ausblendung eines primärstrahls einerseits durch die schlitzförmige Öffnung in der erwähnten drehbaren Blendenplatte und andererseits durch jeweils eine der Öffnungen 33, 43 oder 53 erreicht. Bei der in der Zeichnung dargestellten Ausführungsform würden dazu an sich die Walzen 30, 40 oder 50 ausreichen, doch ist es zweckmäßig, die seitliche Ausdehnung des Primärstrahls 3, 4 oder 5 durch die schlitzförmigen Öffnungen 33, 43 oder 53 begrenzen zu lassen.

## Patentansprüche

1. Anordnung mit räumlichem Auflösungsvermögen zur Untersuchung eines Körpers (6) mit Gamma- oder Röntgenstrahlung, mit einer Strahlenquelle (1), einer zwischen der Strahlenquelle und dem Körper befindlichen Blendenanordnung (z.B. 40) mit einer Öffnung (41, 42) für einen primärstrahl geringen Querschnitts und einer mehrere parallel nebeneinander angeordnete langgestreckte Detektoren umfassenden Detektoranordnung (46, 47), die durch mindestens einen Schlitz (44, 45) hindurch von der der in bestimmten Abschnitten des Körpers durch den primärstrahl erzeugten Streustrahlung getroffen wird, dadurch gekennzeichnet, daß die Blendenanordnung einen drehbaren Blendenteil (40) enthält, der so ausgebildet ist, daß sich bei einer Drehung der primärstrahl (4) in einer Richtung verschiebt, daß die Blendenanordnung und die Detektoranordnung (46, 47) in einem gemeinsamen Gehäuse angeordnet sind, das eine Eintritts- und eine Austrittsöffnung (43) für den Durchtritt des primärstrahls enthält und auf der von der Strahlenquelle abgewandten Seite mit dem Schlitz (44, 45) versehen ist, und daß die Detektoren der Detektoranordnung (46, 47) und der Schlitz (44, 45) parallel zur Verschiebungsrichtung des primärstrahls (4) verlaufen.

2. Anordnung nach Anspruch 1,
dadurch gekennzeichnet, daß die Blendenanordnung (8;30,40,50) mehrere Öffnungen (31,32,33; 41,42,43; 51,52,53) für mehrere primärstrahlen (3,4,5) enthält und daß jedem Primärstrahl wenigstens je ein Schlitz (34,35) und je eine Detektoranordnung zur Erfassung der von dem durch die Öffnung hindurchtretenden Primärstrahl erzeugten Streustrahlung zugeordnet ist.

## Claims

1. An arrangement having a spatial resolution for examining a body (6) by gamma or X-rays, comprising a radiation source (1), a diaphragm device (for example, 40) which is situated between the radiation source and the body and which comprises an aperture (41, 42) for a primary beam of small cross-section, and a detector device (46, 47) which comprises a plurality of adjacently disposed, parallel extending, elongate detectors and which is struck, via at least one slit (44, 45), by the scattered radiation produced by the primary beam in given regions of the body, characterized in that the diaphragm device comprises a rotatable diaphragm portion (40) which is constructed so that in response to a rotation the primary beam (4) is shifted in one direction, the diaphragm device and the detector device (46, 47) being accommodated in a common housing which comprises an entrance aperture and an exit aperture (43) for the passage of the primary beam and which is provided with the slit (44, 45) on the side which is remote from the radiation source, the detectors of the detector device (46, 47) and the slit (44, 45) extending parallel to the direction of shift of the primary beam (4).

2. An arrangement as claimed in Claim 1, characterized in that the diaphragm device (8; 30, 40, 50) comprises a plurality of apertures (31, 32, 33; 41, 42, 43; 51, 52, 53) for a plurality of primary beams (3, 4, 5), with eaoh primary beam there being associated at least one respective slit (34, 35) and one respective detector device for detecting the scattered radiation produced by the primary beam passing through the aperture.

## Revendications

1. Montage à pouvoir de résolution spatiale pour l'examen d'un corps (6) à l'aide d'un rayonnement gamma ou X, comportant une source de rayonnement (1), un dispositif à diaphragmes situé entre la source de rayonnement et le corps (par exemple 40) présentant une ouverture (41, 42) pour un rayonnement primaire de faible section et un dispositif détecteur (46, 47) comportant plusieurs détecteurs oblongs disposés parallèlement côte à côte, qui est frappé, à travers au moins une fente (44, 45), par le rayonnement diffusé produit dans des sections déterminées du corps par le rayonnement primaire, caractérisé en ce que le dispositif à diaphragmes contient une partie à diaphragmes pouvant tourner (40) qui est telle que, lors d'une rotation, le rayonnement primaire (4) se déplace dans un sens, que le dispositif à diaphragmes et le dispositif détecteur (46, 47) sont logés dans un boîtier commun, qui comporte une ouverture d'entrée et une ouverture de sortie (43) permettant le passage du rayonnement primaire et est pourvu de la fente (44, 45) du côté éloigné de la source de rayonnement, et que les détecteurs du dispositif détecteur (46, 47) et la fente (44, 45) s'étendent parallèlement au sens de déplacement du rayonnement primaire (4).

2. Montage suivant la revendication 1, caractérisé en ce que le dispositif à diaphragmes (8; 30, 40, 50) présente plusieurs ouvertures (31, 32, 33; 41, 42, 43; 51, 52; 53) pour plusieurs rayonnements primaires et qu'à chaque rayonnement primaire sont associés au moins une fente (34, 35) et un dispositif détecteur pour capter le rayonnement diffusé produit par le rayonnement primaire traversant l'ouverture.

1/1

FIG. 1

FIG. 2